# EUROPÄISCHE PATENTSCHRIFT

(11) **EP 1 891 024 B1**
(45) Veröffentlichungstag und Bekanntmachung des Hinweises auf die Patenterteilung: **06.10.2010**
(21) Anmeldenummer: 06763407.1
(22) Anmeldetag: 31.05.2006
(51) Int. Cl.: C07D 251/70

(54) **VERFAHREN ZUR HERSTELLUNG VON PULVERFÖRMIGEM ALKOXYCARBONYLAMINOTRIAZIN**
PROCESS FOR THE PREPARATION OF PULVERULENT ALKOXYCARBONYLAMINOTRIAZINE
PROCEDE DE PRODUCTION D'ALCOXYCARBONYLAMINOTRIAZINE SOUS FORME PULVERULENTE

(30) Priorität: 06.06.2005 DE 102005025855
(43) Veröffentlichungstag der Anmeldung: 27.02.2008
(73) Patentinhaber: BASF SE, 67056 Ludwigshafen (DE)
(72) Erfinder: SCHNEIDER, Jörg, B-1970 Wezembeek-oppern (BE); SCHERR, Günter, 67065 Ludwigshafen (DE); EHRHARDT, Rainer, 68199 Mannheim (DE); EICHFELDER, Andreas, 67133 Maxdorf (DE); REIF, Martin, 67354 Römerberg (DE); HIRSCH, Stefan, 67435 Neustadt (DE); SIEDER, Georg, 67098 Bad Dürkheim (DE); HOLTMANN, Thomas, 67346 Speyer (DE); CIPRIAN, Jürgen, 67071 Ludwigshafen (DE); ASCHERL, Hermann, 67246 Dirmstein (DE)
(86) Internationale Anmeldenummer: PCT/EP2006/062764
(87) Internationale Veröffentlichungsnummer: WO 2006/131474

(56) Entgegenhaltungen:
- EP-A- 0 624 577
- WO-A-03/035628
- WO-A-2004/041922
- US-B1- 6 512 026
- DIPL. ING. HEINZ BERGER ET. AL.: "Maschinen und Apparate in der chemischen Industrie" 1968, VEB DEUTSCHER VERLAG FÜR GRUNDSTOFFINDUSTRIE , LEIPZIG, DE , XP002393526 Seite 307 - Seite 318

## Beschreibung

Die Erfindung betrifft ein Verfahren zur Herstellung von pulverförmigem Alkoxycarbonylaminotriazin aus einem alkanolischen, bei der Herstellung von Alkoxycarbonylaminotriazinen anfallenden, mindestens ein Alkoxycarbonylaminotriazin, mindestens einen cyclischen und/oder acyclischen Kohlensäureester, mindestens ein gegebenenfalls ein oder zwei Sauerstoffatome als Etherbindung enthaltendes und gegebenenfalls durch C₁-C₄-Alkyl und/oder Hydroxy substituiertes C₁-C₁₃-Alkanol sowie mindestens ein Alkali- oder Erdalkalialkanolat, gegebenenfalls Melamin und gegebenenfalls Katalysator enthaltenden Reaktionsgemisch.

Die Herstellung von Alkoxycarbonylaminotriazinen durch Umsetzung von Triazinen, beispielsweise Melamin, mit Kohlensäureestern in Gegenwart einer Base ist zum Beispiel aus EP-A 0 624 577 bekannt. Hierbei wird in der Regel Melamin mit einem Kohlensäureester in Gegenwart des dem Kohlensäureester zugrunde liegenden Alkanols und in Gegenwart eines Alkalialkanolats, basierend auf dem dem Kohlensäureester zugrundeliegenden Alkohol, als Base zur Reaktion gebracht. Zur Aufbereitung wird dem Reaktionsgemisch eine mineralische Säure zur Neutralisation zugeführt. Als geeignete Säuren sind Phosphorsäure, Schwefelsäure und/oder Salzsäure genannt. Die Gewinnung des Alkoxycarbonylaminotriazins erfolgt anschließend durch eine Extraktion mit einem organischen Lösemittel und der Verdampfung des Lösemittels. Alternativ wird nach der Zugabe der Säure ein Feststoff durch Filtration isoliert, der dann gewaschen und getrocknet wird.

Aus der WO-A 03/035628 ist ein Verfahren zur Herstellung von Alkoxycarbonylaminotriazinen bekannt, bei welchem die Reaktionsmischung zur Aufbereitung zunächst mit einer bevorzugt wässrigen Säure neutralisiert wird. Als geeignete Säuren sind Salpetersäure, Schwefelsäure, Phosphorsäure oder deren Mischungen, aber auch Ameisensäure genannt. Nach der Zugabe der Säure zum Reaktionsgemisch bilden sich eine wässrige und eine alkanolische Phase, die voneinander getrennt werden. Die alkanolische Phase enthält dabei das Alkoxycarbonylaminotriazin. Zur Erhöhung der Konzentration an Alkoxycarbonylaminotriazin wird die organische Phase nach der Abtrennung der wässrigen Phase eingeengt.

Ein entsprechendes Verfahren zur Aufbereitung eines Alkoxycarbonylaminotriazin enthaltenden Reaktionsgemisches ist auch in WO-A 2004/054990 offenbart.

Aus WO-A 2004/041922 ist ein Herstellungs- und Aufbereitungsverfahren für Carbamat-Melamin-Formaldehyd-Vernetzer bekannt. Die Aufbereitung erfolgt hierbei ebenfalls durch Zugabe einer Säure, z. B. Schwefelsäure, Ameisensäure, Oxalsäure, Phosphorsäure, Salzsäure oder Mischungen daraus. Das bei der Neutralisation entstehende Salz wird durch Filtration und Waschen mit Wasser entfernt.

Ein Verfahren, bei dem ein in n-Butanol gelöstes Tri-Alkoxycarbonylaminotriazin enthaltendes Gemisch zunächst destilliert, dann abgekühlt wird und das so erhaltene Tri-Alkoxycarbonylaminotriazin zu Pulver gemahlen wird, ist aus US 6,512,026 bekannt.

Verschiedene Trocknungsapparate sind z.B. aus Dipl.-Ing. H. Berger, "Maschinen und Apparate in der chemischen Industrie", 1968, VEB Deutscher Verlag für Grundstoffindustrie, Leipzig, Seiten 307-318, bekannt.

Aufgabe der vorliegenden Erfindung ist es, ein Verfahren zur Herstellung von rieselfähigem, pulverförmigen Alkoxycarbonylaminotriazin aus einem alkanolischen, Alkoxycarbonylaminotriazin enthaltenden Reaktionsgemisch bereitzustellen.

Gelöst wird die Aufgabe durch ein Verfahren zur Herstellung von rieselfähigem, pulverförmigem Alkoxycarbonylaminotriazin aus einem alkanolischen, bei der Herstellung von Alkoxycarbonylaminotriazinen anfallenden, mindestens ein Alkoxycarbonylaminotriazin, mindestens einen cyclischen und/oder acyclischen Kohlensäureester, mindestens ein gegebenenfalls ein oder zwei Sauerstoffatome als Etherbindung enthaltendes und gegebenenfalls durch C₁-C₄-Alkyl und/oder Hydroxy substituiertes C₁-C₁₃-Alkanol sowie mindestens ein Alkali- oder Erdalkalialkanolat, gegebenenfalls Melamin und gegebenenfalls Katalysator enthaltenden Reaktionsgemisch, wobei das Reaktionsgemisch in einem Sprühtrockner zerstäubt und getrocknet wird.

Bevorzugte Alkoxycarbonylaminotriazine sind solche der allgemeinen Formel (I) 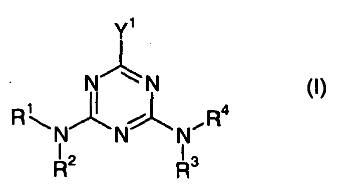 in der die Symbole und Indices die nachfolgende Bedeutung haben:
Y¹ Wasserstoff, C₁-C₄-Alkyl, gegebenenfalls durch C₁-C₄-Alkyl, C₁-C₄-Alkoxy oder Halogen substituiertes Phenyl oder ein Rest der Formel NR⁵R⁶ und
R¹, R², R³, R⁴, R⁵ und R⁶ unabhängig voneinander jeweils Wasserstoff oder ein Rest der Formel COOX oder X oder ausgewählt aus der Gruppe (-CH₂-O)₁-H, (-CH₂-O)ₗ-R, (-CH₂-O)ₖCH₂-N(Z)-Q und (-CH₂-O)ₖ-CH₂-N(Z)-Q, wobei
   - k für 0 bis 10, vorzugsweise 1 bis 5, mehr bevorzugt 1 oder 2 und insbesondere für 1 und I für 1 bis 10, vorzugsweise 1 bis 5, mehr bevorzugt für 1 oder 2 und insbesondere 1 steht,
   - R ausgewählt ist aus der Gruppe Alkyl, Cycloalkyl und Alkylaryl, wobei die Gruppen R vorzugsweise weniger als 13 Kohlenstoffatome enthalten und R bevorzugt ein C₁-C₁₃-Alkyl und besonders bevorzugt Methyl oder Butyl ist,
   - Q ein Triazin-Rest der allgemeinen Formel (II) 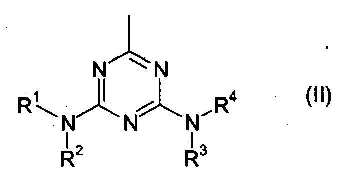 ist,
   - X für C₁-C₁₃-Alkyl, dessen Kohlenstoffgerüst durch 1 oder 2 nicht benachbarte Sauerstoffatome in Etherfunktion unterbrochen und/oder durch Hydroxy substituiert sein kann oder für C₃-C₆-Alkenyl steht und
   - Z für einen Rest R¹, R², R³, R⁴, R⁵ oder R⁶, wie oben definiert, steht
und
mindestens einer der Reste R¹ bis R⁴, oder wenn Y¹ für NR⁵R⁶ steht, mindestens einer der Reste R¹ bis R⁶ COOX.

Dabei bedeutet C₁-C₄-Alkyl z. B. Methyl, Ethyl, Propyl, Isopropyl, Butyl, sec-Butyl oder tert-Butyl.

Gegebenenfalls durch C₁-C₄-Alkyl, C₁-C₄-Alkoxy oder Halogen substituiertes Phenyl sind z. B. Phenyl, 2-, 3- oder 4-Methylphenyl, 2-, 3- oder 4-Ethylphenyl, 2,4-Dimethylphenyl, 2-, 3-oder 4-Methoxyphenyl, 2-, 3- oder 4-Ethoxyphenyl, 2,4-Dimethoxyphenyl, 2-, 3- oder 4-Fluorphenyl oder 2-, 3- oder 4-Chlorphenyl.

C₁-C₁₃-Alkyl, dessen Kohlenstoffgerüst durch 1 oder 2 nicht benachbarte Sauerstoffatome in Etherfunktion unterbrochen und/oder durch Hydroxy substituiert sein kann, bedeutet z. B. Pentyl, Isopentyl, Neopentyl, tert-Pentyl, Hexyl, 2-Methylpentyl, Heptyl, Octyl, 2-Ehtylhexyl, Isooctyl, Nonyl, Isononyl, Decyl, Isodecyl, Undecyl, Dodecyl, Tridecyl, Isotridecyl, 2-Methoxyethyl, 2-Ethoxyethyl, 2-Propoxyethyl, 2-Butoxyethyl, 2- oder 3-Methoxypropyl, 2- oder 3-Ethoxypropyl, 2- oder 3-Propoxypropyl, 2- oder 4-Methoxybutyl, 2- oder 4-Ethoxybutyl, 3,6-Dioxaheptyl, 3,6-Dioxaoctyl, 3,7-Dioxaoctyl, 4,7-Dioxaoctyl, 2-oder 3-Butoxypropyl, 2- oder 4-Butoxybutyl, 2-Hydroxyethyl, 2- oder 3-Hydroxypropyl, 2-oder 4-Hydroxybutyl, 3-Hydroxybut-2-yl. (Die obigen Bezeichnungen Isooctyl, Isononyl, Isodecyl und Isotridecyl sind Trivialbezeichnungen und stammen von den nach der Oxosynthese erhaltenen Alkoholen - vgl. Ullmanns Encyclopedia of Industrial Chemistry, 5th Edition, Vol. A1, Seiten 290 bis 293, sowie Vol. A10, Seiten 284 und 285.)

C₃-C₆-Alkenyl bedeutet z. B. Allyl, Methallyl, Ethallyl, 2-, 3- oder 4-Penten-1-yl oder 2-, 3-, 4- oder 5-Hexen-1-yl.

Gegebenenfalls ein oder zwei nicht benachbarte Sauerstoffatome als Etherbindung enthaltende und gegebenenfalls durch C₁-C₄-Alkyl und/oder Hydroxy-substituiertes C₁-C₁₃-Alkanol sind beispielsweise Methanol, Ethanol, Propanol, Isopropanol, Butanol, Isobutanol, sec-Butanol, tert-Butanol, Pentanol, Isopentanol, Neopentanol, tert-Pentanol, Hexanol, 2-Methylpentanol, Heptanol, Octanol, 2-Ethylhexanol, Isooctanol, Nonanol, Isononanol, Decanol, Isodecanol, Undecanol, Dodecanol, Tridecanol, Isotridecanol, 2-Methoxyethanol, 2-Ethoxyethanol, 2-Propoxyethanol, 2-Butoxyethanol, 2- oder 3-Methoxypropanol, 2- oder 3-Ethoxypropanol, 2- oder 3-Propoxypropanol, 2- oder 4-Methoxybutanol, 2- oder 4-Ethoxybutanol, 3,6-Dioxaheptanol, 3,6-Dioxaoctanol, 3,7-Dioxaoctanol, 4,7-Dioxaoctanol, 2- oder 3-Butoxypropanol, 2- oder 4-Butoxybutanol, Ethan-1,2-diol, Propan-1,2-diol, Propan-1,3-diol, 3-Oxa-5-hydroxypentanol, 3,6-Dioxa-8-hydroxyoctanol, 3-Oxa-5-hydroxy-2,5-dimethylpentanol oder 3,6-Dioxa-8-hydroxy-2,5,8-trimethyloctanol.

Besonders bevorzugt ist das gegebenenfalls ein oder zwei nicht benachbarte Sauerstoffatome als Etherbindung enthaltende und gegebenenfalls durch C₁-C₄-Alkyl und/oder Hydroxy-substituierte C₁-C₁₃-Alkanol ausgewählt aus Ethanol, Propanol, Isopropanol, Butanol, Isobutanol, sec-Butanol, tert-Butanol, Pentanol, Isopentanol, Neopentanol, tert-Pentanol, Hexanol, 2-Methypentanol und Heptanol.

Ganz besonders bevorzugt sind Butanol, Isobutanol, sec-Butanol und tert-Butanol.

Ein cyclischer Kohlensäureester ist ein Carbonat der allgemeinen Formel (III) 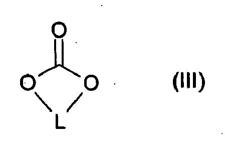 in der

L Ethylen, 1,2- oder 1,3-Propylen oder 1,2-, 1,4-, 2,3- oder 1,3-Butylen bedeutet.

Acyclische Kohlensäureester sind z. B. Diarylcarbonat, Dialkylcarbonat, Arylalkylcarbonat und Dialkenylcarbonat. Bevorzugt ist der acyclische Kohlensäureester ausgewählt aus Carbonaten der allgemeinen Formel (IV)

Z¹O-CO-OZ² (IV)

in der
Z¹ und Z² jeweils unabhängig voneinander Alkyl, Cycloalkyl und Aryl bedeuten. Bevorzugt enthalten die Reste Z¹ und Z² weniger als 13 Kohlenstoffatome. Mehr bevorzugt sind Z¹ und Z² ein C₁-C₈-Alkyl und insbesondere Methyl oder Butyl.

Bevorzugte Dialkylcarbonate sind Dimethylcarbonat, Diethylcarbonat, Dipropylcarbonat, Dibutylcarbonat und Methylbutylcarbonat.

Bevorzugte Arylalkylcarbonate sind Methylphenylcarbonat oder Butylphenylcarbonat.

Geeignete Diarylcarbonate sind z. B. Diphenylcarbonat, Di-(para-tolyl)carbonat, Di-(α-naphtyl)carbonat oder Di-(β-naphtyl)carbonat.

Ein bevorzugtes Dialkenylcarbonat ist Diallylcarbonat.

Besonders bevorzugte Kohlensäureester sind Dimethylcarbonat, Diethylcarbonat, Dibutylcarbonat, Methylbutylcarbonat, Diphenylcarbonat, Propylencarbonat oder Mischungen daraus.

Geeignete Alkali- oder Erdalkalialkanolate sind z. B. Lithium-, Natrium-, Kalium-, Magnesium- oder Calciumsalze der oben näher bezeichneten Alkanole. Die Verwendung von Alkalimethanolaten, insbesondere von Natriummethanolat, ist bevorzugt. Das Alkali- oder Erdalkalialkanolat kann entweder in festem Aggregatzustand oder in gelöster oder suspendierter Form zur Anwendung gelangen.

Bevorzugte LösungsmitteI/Verdünnungsmittel sind in diesem Fall insbesondere die oben näher bezeichneten Alkohole, allein oder als Mischung untereinander. Es können jedoch auch andere an sich bekannte und übliche inerte Verdünnungsmittel zur Anwendung gelangen.

Katalysatoren, die im Reaktionsgemisch enthalten sein können, sind Katalysatoren, die zur Herstellung des Alkoxycarbonylaminotriazins eingesetzt werden. Solche Katalysatoren sind z. B. Phasentransferkatalysatoren, wie sie in Ullmann's Encyclopedia of Industrial Chemistry, 5th Edition, Vol. A19, Seiten 239 bis 248 beschrieben sind. Weitere Katalysatoren können Metallsalze oder -komplexe sein, vorzugsweise Oxide, Chalkogenate, Carbonate oder Halogenide der Alkali-, Erdalkali- oder Übergangsmetalle. Zu nennen sind hier insbesondere Lithiumchlorid, Magnesiumchlorid oder Natriumcarbonat.

Erfindungsgemäß wird aus dem mindestens ein Alkoxycarbonylaminotriazin enthaltenden Gemisch durch eine Sprühtrocknung rieselfähiges, pulverförmiges Alkoxycarbonylaminotriazin erzeugt.

Die Sprühtrocknung erfolgt dabei vorzugsweise in einem Sprühtrockner wie er dem Fachmann bekannt ist. So können für die Sprühtrocknung beispielsweise handelsübliche Sprühtrockner mit Zerstäuberscheibe, Einstoffdüse oder Zweistoffdüse eingesetzt werden. Der Betrieb kann je nach Bauart im Gleichstrom oder im Gegenstrom erfolgen. Bevorzugt wird eine Zweistoffdüse eingesetzt, bei der die flüssige, mindestens ein Alkoxycarbonylaminotriazin enthaltende Phase drucklos mit Hilfe eines Stickstoffstromes zerstäubt wird. Der Stickstoffstrom weist dabei einen Druck im Bereich von 1 bis 10 bar, bevorzugt im Bereich von 2 bis 5 bar und besonders bevorzugt im Bereich von 2,5 bis 5 bar auf. Der Stickstoff wird dabei vorzugsweise als Kreisgas eingesetzt.

Die Sprühtrocknung wird vorzugsweise bei einer Temperatur im Bereich von 50 bis 250°C, bevorzugt bei einer Temperatur von 55 bis 150°C und besonders bevorzugt bei einer Temperatur im Bereich von 60 bis 100°C und bei Umgebungsdruck oder einem Über- oder Unterdruck von bis zu +/- 0,01 MPa, bezogen auf den Umgebungsdruck, durchgeführt.

Das bei der Sprühtrocknung erzeugte, pulverförmige Produkt kann beispielsweise in einem Gewebefilter üblicher Bauart, wie Kerzenfilter, Sackfilter, Schlauchfilter oder anderen dem Fachmann bekannten Filtern oder in einem Zyklon abgetrennt werden. Als Filtermaterial für einen Gewebefilter eignet sich zum Beispiel Polytetrafluorethylen, Silikon oder Polyester. Bevorzugt ist Polyester.

Die Reinigung des als Kreisgas eingesetzten Stickstoffes erfolgt vorzugsweise in einem Wäscher. Dabei ist jeder beliebige, dem Fachmann bekannte Wäscher einsetzbar.

Im Allgemeinen wird das das mindestens eine Alkoxycarbonylaminotriazin enthaltende Reaktionsgemisch aufbereitet, bevor dieses der Sprühtrocknung zugeführt wird. Zur Aufbereitung wird das Gemisch im Allgemeinen in einem ersten Schritt neutralisiert, in einem nächsten Schritt werden ionische und/oder polare Komponenten zum Beispiel durch lonentausch und/oder durch Extraktion entfernt und in einem dritten Schritt kann das Reaktionsgemisch aufkonzentriert werden. Das Aufkonzentrieren kann dabei entweder vor oder nach dem Ionentausch oder vor oder nach der Extraktion erfolgen.

Zum Neutralisieren wird dem alkanolischen Reaktionsgemisch in einer Ausführungsform Säure zugegeben. Die Säure kann dabei konzentriert oder mit Wasser verdünnt zugegeben werden. Eine gleichmäßige Verteilung der Säure im Reaktionsgemisch wird dadurch erreicht, dass während der Dosierung der Säure eine geeignete Durchmischung gewährleistet wird.

In einer weiteren Ausführungsform wird das alkanolische Reaktionsgemisch in eine Säure eingetragen. Die Säure kann dabei konzentriert oder mit Wasser verdünnt sein.

Zum Neutralisieren des Reaktionsgemisches können alle üblichen und industriell verfügbaren organischen und anorganischen Säuren in beliebiger Konzentration, vorzugsweise als 30-85 gew.-%ige wässrige Lösungen, verwendet werden. Vorzugsweise verwendet man Mineralsäuren, deren Salze eine hohe Wasserlöslichkeit aufweisen, wie Salpetersäure, Schwefelsäure oder Phosphorsäure. Eine weitere geeignete Säure ist Ameisensäure. Erfindungsgemäß ist die Verwendung von Salpetersäure besonders bevorzugt.

Zur Abtrennung von ionischen und/oder polaren Komponenten kann die das mindestens eine Alkoxycarbonylaminotriazin enthaltende Phase vor der Sprühtrocknung einem Ionentausch und/oder einer Extraktion zugeführt werden.

Die Extraktion wird mit einem polaren, nicht vollständig mit der organischen Phase mischbaren Extraktionsmittel durchgeführt, wobei eine alkanolische, Alkoxycarbonylaminotriazin enthaltende Phase und eine polare, Extraktionsmittel mit darin gelösten Salzen enthaltende Phase erhalten wird. Nicht vollständig mischbar bedeutet dabei, dass sich zwei Phasen mit unterschiedlicher Zusammensetzung bilden, wobei unter nicht vollständig mischbar auch verstanden wird, dass sich das Extraktionsmittel und die organische Phase überhaupt nicht mischen. Bevorzugt als Extraktionsmittel ist Wasser, besonders bevorzugt ist voll entsalztes Wasser.

Ionische Komponenten entstehen zum Beispiel durch die Neutralisation des alkanolischen Reaktionsgemisches durch Zugabe von Säure.

Die Neutralisation des alkanolischen Reaktionsgemisches und die Entfernung der durch die Neutralisation entstehenden Salze durch Extraktion können in einem Schritt oder in getrennten Verfahrensschritten erfolgen. Bevorzugt erfolgen die Neutralisation und das Entfernen der durch die Neutralisation entstehenden Salze in zwei Schritten.

Für die Extraktion können die dem Fachmann bekannten Apparate, z. B. Mixer/Settler-Einheiten, Kolonnen mit oder ohne Energieeintrag oder Extraktoren, die auf dem Prinzip der Zentrifugalfeldtrennung basieren, eingesetzt werden. Eine Mixer/Settler-Einheit umfasst im Allgemeinen eine Mischeinheit wie einen Rührbehälter, eine Mischpumpe, eine Düse oder einem statischen/dynamischen Mischer. Weiterhin umfasst die Mixer/Settler-Einheit einen Abscheider, der im Allgemeinen als liegender Behälter mit oder ohne Einbauten ausgeführt ist.

Geeignete Kolonnen, die für die Extraktion eingesetzt werden können, sind z. B. Füllkörper-, Packungs- oder Siebbodenkolonnen. Geeignete Siebbodenkolonnen sind z. B. auch Querstromsiebbodenkolonnen. Als Füllkörper können alle dem Fachmann bekannten Füllkörper verwendet werden. Solche Füllkörper sind zum Beispiel in Klaus Sattler, Thermische Trennverfahren, 2. Auflage, VCH Verlagsgesellschaft mbH, Weinheim, 1995, Seiten 226 bis 229 beschrieben.

Als Packungen eignen sich geordnete oder ungeordnete Packungen. Solche Packungen sind z. B. strukturierte Packungen, Lamellenpackungen, Gewebe, Gestricke oder Gewirke.

Die Füllkörper, Packungen oder Siebböden können aus Metall oder Kunststoff gefertigt sein. Aufgrund der guten Benetzungseigenschaften von Metallen wird bei der Wahl der Waschphase als kontinuierliche Phase vorzugsweise Metall als Werkstoff für die Füllkörper, Packungen oder Siebböden verwendet. Besonders geeignete Metalle sind rostfreie Edelstähle.

Neben dem Betrieb der Kolonnen mit Füllkörpern, Packungen oder Böden mit und ohne Pulsation ist auch ein Einsatz ohne Einbauten, z. B. als Sprühkolonne denkbar. Als Beispiele für geeignete, handelsübliche Extraktionskolonnen mit mechanischen Rührsystemen sind Drehscheibenextraktoren, Old-Rushton-Kolonnen, Kühni-Extraktoren, Rührzellenextraktoren, Graesser-Extraktoren zu nennen. Aber auch Zentrifugalextraktoren wie Podbielniak-Extraktoren oder Lurgi-Westfalia-Extraktoren können eingesetzt werden.

Bei der Extraktion zur Entfernung der durch die Neutralisation entstehenden Salze kann die das polare, nicht vollständig mit der organischen Phase mischbare Extraktionsmittel enthaltende Waschphase entweder die kontinuierliche oder die disperse Phase der Extraktion bilden. In einer bevorzugten Form der Extraktion bildet die Waschphase die kontinuierliche Phase.

Neben der Verwendung eines einzelnen Extraktionsapparates ist es auch möglich, die Extraktion in mehreren Apparaten durchzuführen. Hierbei kann auch eine Kombination verschiedener Apparatetypen eingesetzt werden. Eine bevorzugte Kombination bilden dabei eine Mixer/Settler-Einheit und eine Füllkörperkolonne. In einer besonders bevorzugten Ausführungsform wird die Extraktion zur Entfernung von durch die Neutralisation entstehenden Salzen in einer Packungskolonne durchgeführt.

Bei der Extraktion liegt das Phasenverhältnis von polarer zu organischer Phase in einem Bereich von 0,1 bis 2. Bevorzugt ist ein Phasenverhältnis im Bereich von 0,15 bis 1,5, besonders bevorzugt von 0,2 bis 1 und insbesondere von 0,3 bis 0,5.

Bei Verwendung einer Packungskolonne zur Extraktion wird in einer bevorzugten Ausführungsform die polare Phase als Extrakt über den Sumpf der Kolonne abgezogen, das Raffinat - die organische Phase - läuft vorzugsweise als freier Überlauf ab. Der Fremdphasenanteil, d. h. das Alkanol, im Extrakt wird vorzugsweise durch ein Kunststoffgestrick im Sumpf der Kolonne abgetrennt.

Bei Verwendung von Wasser als polarem Extraktionsmittel liegt der Fremdphasenanteil, d. h. der Anteil an nicht gelöstem Wasser, im Raffinat nach der Phasentrennung im Allgemeinen bei etwa 1 Prozent.

Das Raffinat enthält das gewünschte Produkt. Sollte der Anteil an polaren und ionischen Komponenten im Raffinat größer sein als es die erforderliche Produktspezifikation erlaubt, ist es in einer bevorzugten Ausführungsform möglich, das Raffinat in den Feed zurückzuführen. Dabei kann das Raffinat z.B. entweder direkt in den Feedzulauf zur Extraktion eingespeist werden oder in einen Pufferbehälter, aus dem die Extraktion gespeist wird, zurückgeführt werden.

Die polaren und ionischen Komponenten sind z.B. Alkali- oder Erdalkalisalze, Alkali- oder Erdalkalialkanolate, Säure, cyclische oder acyclische Mono- und/oder Diester der Kohlensäure, Alkan(di)ole sowie polare Melaminderivate. Alkandiole sind z.B. Glykol und Propandiol, polare Melaminderivate sind z.B. Melamin, Mono- und Di-Alkoxycarbonylaminotriazine.

Durch die Möglichkeit, Mono- und Di-Alkoxycarbonylaminotriazine durch die Extraktion aus dem Raffinat zu entfernen, ist eine gezielte Einstellung der Verhältnisse verschiedener A-koxycarbonylaminotriazine im Raffinat möglich.

Die Extraktion in der Packungskolonne wird im Allgemeinen als Gegenstromextraktion durchgeführt. In einer besonders bevorzugten Ausführungsform werden hierzu das polare Extraktionsmittel oberhalb der Packung und das alkanolische Reaktionsgemisch unterhalb der Packung zugeführt. Innerhalb der Kolonne strömt so das polare Extraktionsmittel durch die Packung in Richtung des Kolonnensumpfes und das alkanolische Reaktionsgemisch durch die Packung in Richtung des Kolonnenkopfes. In der Packung vermischen sich das alkanolische Reaktionsgemisch und das polare Extraktionsmittel, wobei die im alkanolischen Reaktionsgemisch enthaltenen Salze an das polare Extraktionsmittel abgegeben und so aus dem alkanolischen Reaktionsgemisch entfernt werden.

Die Temperatur, bei welcher die Extraktion durchgeführt wird, liegt vorzugsweise im Bereich von 10 bis 90°C, besonders bevorzugt im Bereich von 15 bis 50°C.

Ein bevorzugter Druck, bei dem die Extraktion durchgeführt wird, ist der Umgebungsdruck. Es ist jedoch auch möglich, die Extraktion bei einem Druck unterhalb des Umgebungsdrucks oder auch bei einem erhöhten Druck durchzuführen. Wenn die Extraktion bei erhöhtem Druck durchgeführt wird, liegt der Druck vorzugsweise im Bereich von 1 bis 10 bar.

Anstelle der Extraktion ist es in einer weiteren Verfahrensvariante möglich, Salze durch Ionenaustausch an einem Kationentauscher und/oder Anionentauscher aus dem Reaktionsgemisch zu entfernen.

In einer Ausführungsform liegen der Kationentauscher und/oder der Anionentauscher als Festbettionentauscher vor. Anstelle des Festbettionentauschers können in einer weiteren Ausführungsform der Kationentauscher und/oder der Anionentauscher auch als Granulat vorliegen.

In einer bevorzugten Ausführungsform werden die Alkalimetall- und/oder Erdalkalimetallionen mit einem Kationentauscher aus dem alkanolischen Reaktionsgemisch entfernt.

Weiterhin ist es möglich, mit einem Anionentauscher die Anionen der Salze zu entfernen. Dies sind zum Beispiel - abhängig von der verwendeten Säure zur Neutralisation - Nitrat-, Sulfat- oder Phosphationen oder auch die Anionen von organischen Säuren, wie Ameisensäure.

Eine Regeneration des beladenen Anionentauschers erfolgt vorzugsweise mit verdünnten, mineralischen Laugen. Besonders geeignet zur Regeneration des Anionentauschers ist 5-25%ige Natronlauge.

Eine Regeneration des Kationentauschers erfolgt vorzugsweise mit verdünnten, mineralischen Säuren. Eine geeignete mineralische Säure ist z. B. 5-30%ige Salzsäure.

Zum Durchlaufen mehrerer Zyklen wird sowohl das Anionentauscherharz als auch das Kationentauscherharz im Allgemeinen mit einem Lösungsvermittler zwischen organischer und polarer Phase vorbehandelt. Hierzu wird der Ionentauscher mit einer Substanz gespült, die eine Polarität aufweist, die zwischen der Polarität der organischen und der polaren Phase liegt und vorzugsweise mit beiden Phasen mischbar ist. Zum Beispiel eignet sich Methanol als Lösungsvermittler bei Butanol als organischer Phase und Wasser als polarer Phase. Neben einer Regeneration des lonentauscherharzes ist auch ein Verwerfen des beladenen Harzes ohne Regeneration denkbar.

Erfindungsgemäß geeignete Anionentauscher sind zum Beispiel stark basische Anionentauscherharze. Bevorzugt sind vernetzte Polystyrol-Harze oder Styrol-Divinyl-benzol-Copolymere mit tertiären oder quartären Aminen als funktionelle Gruppe und OH-Ionen als Austauschionen. Unter Austauschionen sind dabei die Ionen zu verstehen, die an die funktionellen Gruppen gebunden sind und gegen die aus der Flüssigkeit zu entfernenden Ionen ausgetauscht werden. Bei kommerziell erhältlichen Anionentauschern liegen die funktionellen Gruppen im Allgemeinen als Salze vor. Hierbei sind zum Beispiel CI⁻-lonen an die funktionelle Gruppe gebunden. Um den Anionentauscher einsetzen zu können wird dieser in diesem Fall im Allgemeinen zunächst mit NaOH vorbehandelt, um die CI⁻-lonen gegen OH-Ionen zu tauschen. Geeignete, kommerziell erhältliche Anionentauscher sind zum Beispiel Lewatit® MP62, Lewatit® MP64 oder Lewatit® MP 600 WS der Firma Bayer AG oder auch Amberjet® 4200 CL oder Ambersep® 900 OH der Firma Rohm & Haas Co. Zur Entfernung von Nitrationen sind Ambersep® 900 OH und Lewatit® MP 600 WS bevorzugt, besonders bevorzugt ist Ambersep® 900 OH

Geeignete Kationentauscher sind zum Beispiel stark saure Kationentauscherharze auf Basis einer vernetzten Polystyrol-Matrix oder einer Styrol-Divinylbenzol-Copolymer-Matrix und Sulfonsäure als funktioneller Gruppe mit H⁺-Ionen als Austauschionen. Im Allgemeinen liegen die Kationentauscher ebenso wie die Anionentauscher in ihrer Salzform vor, wenn diese in den Handel gelangen. Um den Kationentauscher einsetzen zu können wird dieser dann im Allgemeinen mit einer Säure, z.B. Schwefelsäure, vorbehandelt, um die Kationen des Salzes durch H⁺-Ionen auszutauschen. Kommerziell erhältliche, geeignete Kationentauscher sind zum Beispiel Lewatit® S2528 oder Lewatit MonoPlus® S100 der Firma Bayer AG, Amberlyst® 40 WET und Amberjet® 1500 H der Firma Rohm & Haas Co. sowie Dowex® N306 von Dow Chemical Co. Zur Entfernung von Natriumionen werden z.B. bevorzugt Amberlyst® 40 WET und Amberjet® 1500 H eingesetzt.

Der Kationentauscher und der Anionentauscher können entweder zusammen als Gemisch, einzeln oder in hintereinander geschalteten Schritten oder Stufen eingesetzt werden. Geeignete Kombinationen geeigneter kommerziell erhältlicher Anionentauscher und Kationentauscher sind bei der Entfernung von Nitratsalzen Ambersep® 900 OH oder Amberjet® 4200 als Anionentauscher und Lewatit® S2528 als Kationentauscher. Bevorzugt ist die Kombination von Ambersep® 900 OH und Lewatit® S2528.

Der Kontakt des alkanolischen Reaktionsgemisches mit dem Kationentauscher und/oder Anionentauscher kann z. B. dadurch erfolgen, dass der Kationentauscher und/oder Anionentauscher z. B. in den Reaktor oder in einen Rührbehälter zum Reaktionsgemisch zugegeben werden, oder dadurch, dass das Reaktionsgemisch einen kontinuierlichen Ionentauscher, wobei der Ionentauscher z. B. als Packung in einem Festbett vorliegt, durchströmt.

Die Zugabe des lonentauscherharzes in den Reaktionsbehälter ist insbesondere dann möglich, wenn das Alkoxycarbonylaminotriazin diskontinuierlich hergestellt wird. In diesem Fall erfolgen bevorzugt sowohl die Herstellung als auch die Neutralisation und die Entfernung der durch die Neutralisationen entstehenden Salze durch Ionentausch im gleichen Behälter.

In einer besonders bevorzugten Ausführungsform wird ein Teil der Salze vor dem lonenaustausch durch Waschen, Extraktion oder Filtration oder Kombinationen daraus aus dem alkanolischen Reaktionsgemisch entfernt.

Das Waschen erfolgt vorzugsweise durch Zugabe von Wasser bei einer Temperatur im Bereich von 10 bis 70°C, bevorzugt von 15 bis 50°C und bei einem pH-Wert von 0 bis 8, bevorzugt von 2 bis 5.

Wird nach dem Ionenaustausch eine Extraktion durchgeführt, wird diese vorzugsweise wie oben beschrieben durchgeführt.

In einem weiteren Schritt kann das alkanolische, mindestens ein Alkoxycarbonylaminotriazin enthaltende Reaktionsgemisch aufkonzentriert werden, bevor dieses der Sprühtrocknung zugeführt wird.

Das Aufkonzentrieren kann dabei durch thermische oder mechanische Verfahren erfolgen. Geeignete thermische Verfahren zum Aufkonzentrieren sind zum Beispiel Verdampfung, Destillation oder Rektifikation. Geeignete mechanische Verfahren sind insbesondere Membrantrennverfahren, zum Beispiel Pervaporation oder Permeation sowie Filtration, wenn das mindestens eine Alkoxycarbonylaminotriazin als Suspension vorliegt. Die Verfahren zum Aufkonzentrieren können jeweils einzeln oder in Kombination zur Anwendung kommen. Es kann auch jedes weitere dem Fachmann bekannte Verfahren zur Aufkonzentrierung eingesetzt werden. Ein bevorzugtes Verfahren zur Aufkonzentrierung ist die Destillation.

Das Aufkonzentrieren der organischen, Alkooycarbonylaminotriazin enthaltenden Phase durch Destillation kann kontinuierlich oder diskontinuierlich erfolgen.

Zur kontinuierlichen Destillation können herkömmliche, dem Fachmann bekannte kontinuierliche Verdampfer eingesetzt werden. Geeignete Verdampfer zur kontinuierlichen Destillation sind z. B. Umlaufverdampfer, wie Robert-Selbstumlaufverdampfer, Schnellumlaufverdampfer mit schrägen Verdampferrohren, Zwangsumlaufverdampfer mit außen liegendem Verdampferbündel, Umlaufverdampfer mit in Kammern unterteiltem Siederaum oder Zwangsumlaufverdampfer mit liegendem Heizkörper. Weitere geeignete kontinuierliche Verdampfer sind z. B. Fallfilmverdampfer, Dünnschichtverdampfer oder Kestnerverdampfer.

Weiterhin kann das Aufkonzentrieren der organischen Phase durch Destillation in einer Kolonne erfolgen. Die Erwärmung auf Verdampfungstemperatur kann dabei am Kolonnensumpf erfolgen oder in einem außerhalb der Kolonne liegenden Wärmetauscher. Geeignete Kolonnen sind z. B. Packungskolonnen, Füllkörperkolonnen oder Bodenkolonnen. Geeignete Packungen, Füllkörper oder Böden sind dabei alle dem Fachmann bekannten Packungen, Füllkörper oder Böden.

Eine diskontinuierliche Aufkonzentrierung durch Destillation kann z. B. in einem Rührbehälter erfolgen. Dabei kann die Destillation auch in dem Behälter durchgeführt werden, in dem die Reaktion zu Alkoxycarbonylaminotriazin durchgeführt wird. Bevorzugt erfolgt die Aufkonzentrierung durch Destillation in einem zusätzlichen Rührbehälter.

Sowohl beim kontinuierlichen als auch beim diskontinuierlichen Verfahren fallen der Alkoxycarbonylaminotriazin enthaltende Produktstrom als flüssige Phase und ein mindestens ein Alkanol, Carbonat und gegebenenfalls Wasser enthaltender Brüdenstrom an. Bei Verwendung eines von Wasser unterschiedlichen polaren Extraktionsmittels bei der Entfernung von durch, die Neutralisation entstehenden Salzen durch Extraktion ist im Brüden entweder anstelle des Wassers oder zusätzlich das polare Extraktionsmittel enthalten.

Die Aufkonzentrierung der organischen, Alkoxycarbonylaminotriazin enthaltenden Phase durch Destillation führt in einer besonders bevorzugten Ausführungsform zu einem Produktstrom, welcher 45-60 Gew.-% Alkoxycarbonylaminotriazin enthält.

Abhängig vom gewünschten Produktstrom ist es jedoch auch möglich, durch die Destillation einen Produktstrom zu erhalten, der einen kleineren oder auch einen größeren Anteil an Alkoxycarbonylaminotriazin enthält.

In einer Verfahrensvariante werden der bei der Sprühtrocknung anfallende Brüdenstrom und gegebenenfalls der bei der Aufkonzentrierung des Alkoxycarbonylaminotriazins anfallende an Alkoxycarbonylaminotriazin abgereicherte Strom destillativ in eine organische Phase und eine polare Phase getrennt. Die Trennung kann dabei kontinuierlich oder diskontinuierlich erfolgen.

Bevor der Brüdenstrom der destillativen Trennung zugeführt wird, kann dieser in einer bevorzugten Verfahrensvariante kondensiert werden. Es ist jedoch auch möglich, den Brüden dampfförmig der destillativen Trennung zuzuführen.

Wird die destillative Trennung kontinuierlich durchgeführt, erfolgt dies vorzugsweise in einer Kolonne. Geeignete Kolonnen sind Packungskolonnen, Füllkörperkolonnen oder Bodenkolonnen. Als Packungen eignen sich z. B. geordnete Packungen oder Gestricke oder Gewirke. Bei Einsatz einer Füllkörperkolonne geeignete Füllkörper sind dem Fachmann bekannt und z. B. in Klaus Sattler, Thermische Trennverfahren, 2. Auflage, VCH Verlagsgesellschaft mbH, Weinheim, 1995, Seiten 226 bis 229 offenbart.

Bei Einsatz einer Bodenkolonne geeignete Böden sind z. B. Siebböden, Glockenböden, Tunnelböden oder Kreuzstromböden.

Der aufgefangene, vorzugsweise kondensierte Brüdenstrom kann der Kolonne entweder am Kopf, im Sumpf oder über einen Seitenzulauf zugeführt werden. Bevorzugt wird der Brüdenstrom über einen Seitenzulauf zugeführt.

Durch die destillative Trennung wird am Kolonnensumpf ein im Wesentlichen wasserfreies Gemisch aus Carbonat und Alkanol und am Kolonnenkopf ein carbonatfreies Gemisch aus leichtsiedenden Alkanolen und gegebenenfalls Wasser und/oder polarem Extraktionsmittel erhalten.

Eine Verbesserung der Alkanolrückgewinnung kann dadurch erreicht werden, dass die über den Kopf der Kolonne abgezogene leichtsiedende Phase einem Phasenscheider zugeführt wird. Im Phasenscheider erfolgt eine Auftrennung in eine im Wesentlichen Alkanol enthaltende Phase und eine im Wesentlichen Wasser und/oder polare Extraktionsmittel enthaltende Phase. Ein weiterer Vorteil des Einsatzes eines Phasenscheiders ist, dass der Energiebedarf für Verdampfung und Kondensation verringert wird, dass die Kolonnenbelastung verringert wird und weiterhin, dass der Carbonatverlust reduziert wird.

Die bei der Phasentrennung gewonnene, im Wesentlichen Alkanol enthaltende Phase aus dem Phasenscheider wird vorzugsweise als Rücklauf in die Kolonne zurückgeführt. Ein Vorteil der Rückführung der alkanolischen Phase ist, dass die Trennstufenzahl geringer ist als bei einer Kolonne ohne Rücklauf aus dem Phasenscheider und somit die Kolonnenhöhe reduziert werden kann.

In einer weiteren Verfahrensvariante wird der auskondensierte Brüdenstrom einem Phasenscheider zugeführt. Im Phasenscheider erfolgt eine Auftrennung in eine im Wesentlichen organische Phase und eine im Wesentlichen polare Phase. Die im Wesentlichen organische Phase wird der Destillationskolonne zugeführt. In der Destillationskolonne erfolgt eine weitere Auftrennung in ein Carbonat und Alkanol enthaltendes Sumpfprodukt sowie ein leichtsiedendes, Alkanol und gegebenenfalls Wasser und/oder polares Extraktionsmittel enthaltendes Kopfprodukt.

Vorteil gegenüber der Verfahrensvariante, bei welcher der der Destillationskolonne entnommene Kopfstrom einem Phasenscheider zugeführt wird, ist, dass die Feedmenge und der Wasseranteil im Feed stark reduziert sind und somit eine Kolonne mit einem geringeren Kolonnendurchmesser eingesetzt werden kann und der Energiebedarf reduziert ist.

Besonders bevorzugt zur kontinuierlichen destillativen Trennung ist die Verfahrensvariante, bei welcher der über den Kopf der Destillationskolonne abgezogene, Wasser und/oder polares Extraktionsmittel und leichtsiedende Alkanole enthaltende Strom einem Phasenscheider zugeführt wird. Das Rücklaufverhältnis in der Destillationskolonne liegt vorzugsweise in einem Bereich zwischen 0,3 und 2,8 kg/kg. Die Destillation wird vorzugsweise in einer Destillationskolonne mit 8 bis 18 theoretischen Trennstufen durchgeführt.

Der Druck, bei welchem die Destillation durchgeführt wird, liegt vorzugsweise im Bereich zwischen 0,5 und 2000 mbar, besonders bevorzugt zwischen 50 und 950 mbar.

Bei der diskontinuierlichen destillativen Trennung erfolgt im Unterschied zur kontinuierlichen destillativen Trennung kein Auffangen des Brüdenstromes, sondern die einzelnen Brüdenströme bzw. Fraktionen werden sequentiell zur Aufkonzentrierung durch Destillation abdestilliert. Hierbei kann die eingesetzte Destillationskolonne als reine Verstärkungskolonne oder als Destillationskolonne mit Verstärkungs- und Abtriebsteil betrieben werden.

Wenn die Kolonne als reine Verstärkungskolonne betrieben wird, befindet sich der Brüdenzulauf vorzugsweise als Seitenzulauf im Kolonnensumpf. Bei Betrieb der Destillationskolonne als reine Verstärkungskolonne werden alle Fraktionen nacheinander als Destillat abgezogen. Als erste Fraktion wird ein Gemisch aus leichtsiedendem Alkanol und Wasser und/oder polarem Extraktionsmittel als Kopfprodukt abgezogen. Dieses Gemisch wird in einem Phasenscheider in eine organische und eine polare Phase getrennt. In einer bevorzugten Ausführungsform wird die organische Phase anschließend wieder dem Kopf der Kolonne zugeführt. Die polare Phase wird ausgeschleust.

Sobald die Menge der ausgeschleusten polaren Phase einen vorgegebenen Wert unterschreitet, wird das Destillat in einem Zwischenbehälter aufgefangen. Die Wasserkonzentration bzw. Konzentration an polarem Extraktionsmittel im Destillat, welches dem Zwischenbehälter zugeführt wird, wird gemessen. Sobald die Konzentration an Wasser bzw. polarem Extraktionsmittel einen vorgegebenen Wert unterschreitet, wird der Zulauf zum Zwischenbehälter verschlossen und die schwer siedende Fraktion, Carbonat und schwersiedende Alkanole enthaltend, ausgeschleust.

Wenn die Zusammensetzung des Destillatstromes nach dem Abtrennen der leichtsiedenden Fraktion bereits die erforderliche Zusammensetzung aufweist, ist es selbstverständlich möglich, auf den Zwischenbehälter zu verzichten und direkt die schwersiedende Fraktion auszuschleusen. Vorteil des Betriebs der Destillationskolonne als reine Verstärkungskolonne ist deren geringe Kolonnenhöhe.

Der Vorteil des Betriebs der Destillationskolonne mit Abtriebs- und Verstärkungsteil ist die höhere Rückgewinnungsrate in Bezug auf die Carbonate sowie ein geringerer Energieverbrauch und eine geringere Destillationszeit.

In einer bevorzugten Ausführungsform wird beim Betrieb der Destillationskolonne als reine Verstärkungskolonne der Sumpf der Kolonne zurück in den Apparat geführt, in welchem die organische, Alkoxycarbonylaminotriazin enthaltende Phase durch Destillation aufkonzentriert wird.

Die bevorzugte Verfahrensvariante bei der diskontinuierlichen destillativen Trennung ist die, bei welcher die Destillationskolonne als Destillationskolonne mit Verstärkungs- und Abtriebsteil betrieben wird. Der Vorteil dieser Verfahrensvariante sind die höheren Rückgewinnungsraten für Carbonate als beim Betrieb der Destillationskolonne als reine Verstärkungskolonne.

Beim Betrieb der Destillationskolonne als Destillationskolonne mit Verstärkungs- und Abtriebsteil wird der aus dem Sprühtrockner anfallende Brüden direkt als Seitenzulauf einer Destillationskolonne zugeführt. Innerhalb der Destillationskolonne erfolgt eine Trennung in eine leichtsiedende und eine schwersiedende Fraktion. Bei der destillativen Trennung wird zunächst in einem Vorlauf ein Gemisch, welches leichtsiedendes Alkanol sowie Wasser und/oder polares Extraktionsmittel enthält, als Kopfprodukt abgezogen. In einer bevorzugten Ausführungsform wird der Vorlauf in einem Phasenscheider in eine organische und eine polare Phase getrennt. Die organische Phase wird vorzugsweise wieder als Vorlauf dem Kopf der Destillationskolonne zugeführt und die polare Phase ausgeschleust.

Sobald die abgezogene polare Phase einen vorgegebenen Wert unterschreitet, wird das Destillat in einem Zwischenbehälter aufgefangen. Dabei wird das Destillat, welches im Zwischenbehälter aufgefangen wird, in einer bevorzugten Ausführungsform nicht über einen Phasenscheider geführt.

Sobald die Konzentration an Wasser bzw. polarem Extraktionsmittel im Destillat einen vorgegebenen Wert unterschreitet, wird der Zulauf zum Zwischenbehälter verschlossen und die schwer siedende Fraktion aus dem Sumpf der Destillationskolonne ausgeschleust. Ein Rücklauf in den Apparat, in welchem die organische, Akoxycarbonylaminotriazin enthaltende Phase aufkonzentriert wird, erfolgt dann nicht mehr.

Das Rücklaufverhältnis liegt vorzugsweise im Bereich zwischen 0 und 15 kg/kg.

Eine bevorzugte Destillationskolonne bei der diskontinuierlich durchgeführten destillativen Trennung weist zwischen 4 und 10 theoretische Trennstufen auf. Die Kolonne wird - wie auch bei der kontinuierlichen destillativen Trennung - vorzugsweise bei einem Druck im Bereich von 0,5 bis 2000 mbar am Kopf der Kolonne betrieben. Besonders bevorzugt liegt der Druck zwischen 50 und 950 mbar.

Über den Kopf der Destillationskolonne werden Alkanole, gegebenenfalls Leichtsieder und Wasser und/oder polares Extraktionsmittel abgezogen. Diese Strom wird in einer besonders bevorzugten Ausführungsform anschließend einem Phasenscheider zugeführt, in welchem die polare Phase von der organischen Phase getrennt wird. Die organische Phase wird vorzugsweise als Rücklauf erneut der Destillationskolonne an deren Kopf zugeführt.

In einer besonders bevorzugten Ausführungsform umfasst die Destillationskolonne einen vorzugsweise im Abtriebsteil angeordneten Seitenabzug, über welchen ein vorzugsweise dampfförmiger und im Wesentlichen wasserfreier, Carbonat und Alkanol enthaltender Strom abgezogen wird. Eine besonders bevorzugte Position des Seitenabzuges ist direkt oberhalb des Kolonnensumpfes bzw. direkt unterhalb der trennwirksamen Einbauten in der Kolonne.

Vorteile dieser Betriebsweise liegen in der Rückgewinnung eines Großteils der Carbonate über Seitenabzug zum Wiedereinsatz in der Reaktion, der Reduzierung des Carbonatgehaltes im Produktstrom und in der Rückgewinnung von wasserfreien schwersiedenden Alkanolen, z.B. n-Butanol, über den Seitenabzug.

In einer bevorzugten Ausführungsform umfasst die Destillationskolonne 8 bis 22 theoretische Trennstufen.

Das Rücklaufverhältnis der organischen Phase am Kopf der Kolonne liegt vorzugsweise im Bereich zwischen 0,2 und 3 kg/kg.

### Beispiele

### Beispiel 1

30 kg/h einer Mischung aus 50% Trialkoxycarbonylaminotriazin und 50% Butanol wurden in einen Sprühturm mit einer Zweistoffdüse mit einem Durchmesser von 6 mm zerstäubt. Als Gas zur Zerstäubung wurde Stickstoff mit einem Druck von 4,5 bar und einem Massenstrom von 80 kg/h eingesetzt. Die Mischung aus Trialkoxycarbonylaminotriazin und Butanol wurde der Zweistoffdüse drucklos zugeführt. Die Trocknung im Sprühtrockner erfolgte unter Stickstoffatmosphäre. Hierzu wurde der Sprühtrockner mit 1480 kg/h Stickstoff als Kreisgas mit einer Gaseintrittstemperatur von 91°C und einer Gasaustrittstemperatur von 76°C bei Umgebungsdruck betrieben. Im Sprühtrockner entstand ein weißes Pulver.

### Beispiel 2

8g/min einer Mischung aus 50% Trialkoxycarbonylaminotriazin und 50% Butanol wurden in einen Sprühturm mit einer Zweistoffdüse mit einem Durchmesser von 0,5 mm zerstäubt. Als Gas zur Zerstäubung wurden der Zweistoffdüse 800l/h Stickstoff zugeführt. Der Sprühtrockner wurde mit einem Überdruckruck von 18 mbar betrieben. Die Trocknung im Sprühtrockner erfolgte unter Stickstoffatmosphäre. Hierzu wurde der Sprühtrockner mit 10 m³/h Stickstoff mit einer Gaseintrittstemperatur von 126°C und einer Gasaustrittstemperatur von 73°C als Kreisgas betrieben. Im Sprühtrockner entstand ein weißes Pulver.

## Patentansprüche

1. Verfahren zur Herstellung von rieselfähigem, pulverförmigem Alkoxycarbonylaminotriazin aus einem alkanolischen, bei der Herstellung von Alkoxycarbonylaminotriazinen anfallenden, mindestens ein Alkoxycarbonylaminotriazin, mindestens einen cyclischen und/oder acyclischen Kohlensäureester, mindestens ein gegebenenfalls ein oder zwei Sauerstoffatome als Etherbindung enthaltendes und gegebenenfalls durch C₁-C₄-Alkyl und/oder Hydroxy substituiertes C₁-C₁₃-Alkanol sowie mindestens ein Alkali- oder Erdalkalialkanolat, gegebenenfalls Melamin und gegebenenfalls Katalysator enthaltenden Reaktionsgemisch, wobei das Reaktionsgemisch in einem Sprühtrockner zerstäubt und getrocknet wird.

2. Verfahren nach Anspruch 1, **dadurch gekennzeichnet, dass** die Sprühtrocknung bei einer Temperatur im Bereich von 50 bis 250°C und bei Umgebungsdruck oder einem Über- oder Unterdruck von bis zu +/- 0,01 MPa, bezogen auf den Umgebungsdruck, durchgeführt wird.

3. Verfahren nach Anspruch 1 oder 2, **dadurch gekennzeichnet, dass** die Sprühtrocknung bei einer Temperatur im Bereich von 70 bis 100°C durchgeführt wird.

4. Verfahren nach einem der Ansprüche 1 bis 3, **dadurch gekennzeichnet, dass** das pulverförmige Alkoxycarbonylaminotriazin in einem Gewebefilter oder einem Zyklon abgetrennt wird.

5. Verfahren nach Anspruch 4, **dadurch gekennzeichnet, dass** der Gewebefilter ein Kerzenfilter, Sackfilter oder Schlauchfilter ist.

6. Verfahren nach Anspruch 4 oder 5, **dadurch gekennzeichnet, dass** das Filtermaterial ausgewählt ist aus Polytetrafluorethylen, Silikon und Polyester.

7. Verfahren nach einem der Ansprüche 1 bis 6, **dadurch gekennzeichnet, dass** das das mindestens eine Alkoxycarbonylaminotriazin enthaltende Reaktionsgemisch mit Hilfe eines Stickstoffstromes zerstäubt wird.

8. Verfahren nach einem der Ansprüche 1 bis 7, **dadurch gekennzeichnet, dass** die Sprühtrocknung in einer Stickstoffatmosphäre durchgeführt wird.

9. Verfahren nach Anspruch 8, **dadurch gekennzeichnet, dass** der Stickstoff als Kreisgas eingesetzt wird.

10. Verfahren nach Anspruch 9, **dadurch gekennzeichnet, dass** der als Kreisgas eingesetzte Stickstoff in einem Wäscher gereinigt wird.

11. Verfahren nach einem der Ansprüche 1 bis 10, **dadurch gekennzeichnet, dass** das das mindestens eine Alkoxycarbonylaminotriazin enthaltende Reaktionsgemisch vor der Sprühtrocknung aufgearbeitet wird, wobei das Gemisch in einem ersten Schritt neutralisiert wird, in einem nächsten Schritt ionische und/oder polare Komponenten durch Ionenaustausch und/oder Extraktion entfernt werden und das Gemisch gegebenenfalls in einem dritten Schritt aufkonzentriert wird.

12. Verfahren nach Anspruch 11, **dadurch gekennzeichnet, dass** das Aufkonzentrieren vor oder nach dem Ionentausch oder vor oder nach der Extraktion erfolgt.

13. Verfahren nach Anspruch 11 oder 12, **dadurch gekennzeichnet, dass** das Aufkonzentrieren durch Verdampfung, Destillation, Rektifikation oder Membrantrennverfahren erfolgt.

14. Verfahren nach einem der Ansprüche 1 bis 13, **dadurch gekennzeichnet, dass** der bei der Sprühtrocknung anfallende Brüdenstrom und gegebenenfalls ein bei der Aufkonzentrierung des Alkoxycarbonylaminotriazins anfallender, an Alkoxycarbonylaminotriazin abgereicherter Strom destillativ in eine organische und eine polare Phase getrennt werden.

## Claims

1. A process for prepaying free-flowing, pulverulent alkoxycarbonylaminotriazine from an alkanolic redaction mixture which is obtained in the preparation of alkoxycarbonylaminotriazines and comprised at least one alkoxycarbonylaminotriazine, at least one cyclic and/or acyclic carbonic ester, at least one C₁-C₁₃-alkanol which optionally comprises one or two oxygen atoms in the form of ether bounds and is optionally substituted by C₁-C₄-alkyl and/or hydroxy, and also at least one alkali metal or alkaline earth metal alkoxide, with or without melamine and with or without catalyst, by atomizing and dying the reaction mixture in a spray drier.

2. The process according to claim 1, wherein the spray dying is performed at a temperature in the range from 50 to 250°C and at ambient pressure or an elevated or reduced pressure of up to +/-0.01 MPa based on the ambient pressure.

3. The process according to claim 1 or 2, wherein the spray drying is carried oust at a temperature in the range from 70 to 100°C.

4. The process according to any of claims 1 to 3, wherein the pulverulent alkoxycarbonylaminotriazine is removed in a fabric filter or a cyclone.

5. The process according to claim 4, wherein the fabric filter is a candle filler, bag filter or hose filter.

6. The process according to claim 4 oar 5, wherein the filter material is selected from polytetrafluoroethylene, silicone and polyester,

7. The process according to any of claims 1 to 6, wherein the reaction mixture comprising at least one alkoxycarbonylaminotriazine is atomized with the aid of a nitrogen stream.

8. The process according to any of claims 1 to 7, wherein the spray drying is performed in a nitrogen atmosphere.

9. The process according to claim 8, wherein the nitrogen is used as cycle gas.

10. The process according to claim 9, wherein the nitrogen used as cycle gas is purified in a scrubber.

11. The process according to any of claims 1 to 10, wherein the reaction mixture comprising the at least one alkoxycarbonylaminotriazine is worked up before the spray drying, by neutralizing the mixture in a first step, removing ionic and/or polar components by ion exchange and/or extraction in a next step and concentrating the mixture, if appropriate, in a third step.

12. The process according to claim 11, wherein the concentration is effected before or after the in exchange or before or after the extraction.

13. The process according to claim 11 or 12, wherein the concentration is effected by evaporation, distillation, rectification or membrane reparation processes.

14. The process according to any of claims 1 to 13, wherein the vapor stream obtained in the spray drying and, if appropriate, a stream which has been depleted in alkoxycarbonylaminotriazine and is obtained in the concentration of the alkoxycarbonylaminotriazine is separated distillatively into an organic and a polar phase.

## Revendications

1. Procédé de fabrication d'alcoxycarbonylaminotriazine pulvérulente fluide à partir d'un mélange réactionnel alcanolique, formé lors de la fabrication d'alcoxycarbonylaminotriazines, contenant au moins une alcoxycarbonylaminotriazine, au moins un ester d'acide carbonique cyclique et/ou acyclique, au moins un alcanol en C₁-C₁₃ éventuellement substitué par un alkyle en C₁-C₄ et/ou par un hydroxy et contenant éventuellement un ou deux atomes d'oxygène sous la forme d'une liaison éther, ainsi qu'au moins un alcanolate alcalin ou alcalino-terreux, éventuellement de la mélamine et éventuellement un catalyseur, le mélange réactionnel étant pulvérisé et séché dans un séchoir à pulvérisation.

2. Procédé selon la revendication 1, **caractérisé en ce que** le séchage par pulvérisation est réalisé à une température dans la plage allant de 50 à 250 °C et à la pression ambiante ou à une sur- ou sous-pression pouvant atteindre ± 0,01 MPa par rapport à la pression ambiante.

3. Procédé selon la revendication 1 ou 2, **caractérisé en ce que** le séchage par pulvérisation est réalisé à une température dans la plage allant de 70 à 100 °C

4. Procédé selon l'une quelconque des revendications 1 à 3, **caractérisé en ce que** l'alcoxycarbonylaminotriazine pulvérulente est séparée dans un tissu filtrant ou dans un cyclone.

5. Procédé selon la revendication 4, **caractérisé en ce que** le tissu filtrant est un filtre à bougie, un filtre à sac ou un filtre à manche.

6. Procédé selon la revendication 4 ou 5, **caractérisé en ce que** le matériau de filtration est choisi parmi le polytétrafluoroéthylène, la silicone et le polyester.

7. Procédé selon l'une quelconque des revendications 1 à 6, **caractérisé en ce que** le mélange réactionnel contenant la ou les alcoxycarbonylaminotriazines est pulvérisé à l'aide d'un courant d'azote.

8. Procédé selon l'une quelconque des revendications 1 à 7, **caractérisé en ce que** le séchage par pulvérisation est réalisé dans une atmosphère d'azote.

9. Procédé selon la revendication 8, **caractérisé en ce que** l'azote est utilisé sous la forme d'un gaz circulaire.

10. Procédé selon la revendication 9, **caractérisé en ce que** l'azote utilisé sous la forme d'un gaz circulaire est purifié dans un épurateur.

11. Procédé selon l'une quelconque des revendications 1 à 10, **caractérisé en ce que** le mélange rédactionnel contenant la ou les alcoxycarbonylaminotriazines est traité avant le séchage par pulvérisation, le mélange étant neutralisé lors d'une première étape, les composants ioniques et/ou polaires étant éliminés par échange d'ions et/ou extraction lors d'une étape ultérieure et le mélange étant éventuellement concentré lors d'une troisième étape.

12. Procédé selon la revendication 11, **caractérisé en ce que** la concentration a lieu avant ou après l'échange d'ions ou avant ou auprès l'extraction.

13. Procédé selon la revendication 11 ou 12, **caractérisé en ce que** la concentration a lieu par un procédé d'évaporation, de distillation, de rectification ou de séparation sur membrane.

14. Procédé selon l'une quelconque des revendications 1 à 13, **caractérisé en ce que** le courant de vapeur formé lors du séchage par pulvérisation et éventuellement un courant appauvri en alcoxycarbonylaminotriazine formé lors de la concentration de l'alcoxycarbonylaminotriazine sont séparés par distillation en une phase organique et une phase polaire.
